# EUROPEAN PATENT SPECIFICATION

(11) **EP 2 897 685 B1**
(45) Date of publication and mention of the grant of the patent: **18.08.2021**
(21) Application number: 13838419.3
(22) Date of filing: 17.05.2013
(51) Int. Cl.: A61N 1/375

(54) **EXTRACELLULAR MATRIX ENCASEMENT STRUCTURES AND METHODS**
GEHÄUSESTRUKTUREN UND VERFAHREN FÜR EXTRAZELLULÄRE MATRIX
STRUCTURES ET MÉTHODES DE CONFINEMENT DE MATRICE EXTRACELLULAIRE

(30) Priority: 24.09.2012 US 201213573566
(43) Date of publication of application: 29.07.2015
(73) Proprietor: Aziyo Med, LLC, Silver Spring, MD 20904 (US)
(72) Inventor: MATHENY, Robert, G., Norcross, GA 30092 (US)
(74) Representative: HGF
(86) International application number: PCT/US2013/041517
(87) International publication number: WO 2014/046741

(56) References cited:
- WO-A1-2012/018680
- WO-A1-2012/018680
- US-A1- 2009 138 074
- US-A1- 2010 233 235
- US-A1- 2010 233 235
- None

## Description

### FIELD OF THE INVENTION

The present invention relates to implantable structures and devices; particularly, medical devices, encased in extracellular matrix (ECM) based pouches in accordance with the claims that effectuate modulated healing of damaged tissue and regeneration of new tissue structures with site-specific structural and functional properties.

### BACKGROUND OF THE INVENTION

As is well known in the art, treatment of various medical conditions commonly involves implantation of medical devices and/or insertion of medical instruments into a body. Illustrative is the implantation or deployment of heart valves to regulate the flow of blood through cardiovascular vessels, and pacemakers to control abnormal heart rhythms.

Implantable medical devices; particularly, cardiovascular implants, have unique blood biocompatibility requirements to ensure that the device is not rejected (as in the case of natural tissue materials for heart valves and grafts for heart transplants) or that adverse thrombogenic (clotting) or hemodynamic (blood flow) responses are avoided.

Several cardiovascular implants, such as heart valves, are formed from natural tissue. Illustrative are the heart valves disclosed in U.S. Pat. Nos. 6,719,788 and 5,480,424 to Cox. The disclosed bioprostheses can, however, be affected by gradual calcification, which can, and in many instances will, lead to the eventual stiffening and tearing of the implant.

Many non-bioprosthetic implants are, however, fabricated from various metals and polymeric materials, and other exotic materials, such as pyrolytic carbon-coated graphite.

For example, pacemakers, defibrillators, leads, and other similar cardiovascular implants are often fabricated from Ni--Co--Cr alloy, Co--Cr--Mo alloy, titanium, and Ti-6Al-4V alloy, stainless steel, and various biocompatible polymeric materials. Artificial heart valves are often fabricated from various combinations of nylon, silicone, titanium, Teflon™, polyacetal, graphite and pyrolytic carbon.

Artificial hearts and ventricular assist devices are often fabricated from various combinations of stainless steel, cobalt alloy, titanium, Ti-6Al-4V alloy, carbon fiber reinforced composites, polyurethanes, Biolon™, Hemothane™, Dacron™, polysulfone, and other thermoplastics.

Finally, catheters and guide wires are often fabricated from Co--Ni or stainless steel wire. In many instances, the wire is encased in a polymeric material.

As is well known in the art, several major problems are often encountered when a medical device (or other device, e.g., tracking apparatus) fabricated from one of the aforementioned materials is implanted in the body. A major problem that is often encountered after implantation of such a device in the body is inflammation of surrounding tissue.

Another major problem is the high incidence of infection.

A further problem that is often encountered after implantation of the medical device in the body is the formation of blood clots (thrombogenesis).

One additional problem that is also often encountered is the degradation, e.g., corrosion, of medical device leads and, thereby, premature failure of the device after implantation in the body.

Most medical devices are designed to be implanted in the body for an extended period of time. However, when a harsh biological response (or premature failure of the device) is encountered after implantation, it is often necessary to remove the device through a secondary surgical procedure, which can, and in many instances will, result in undesirable pain and discomfort to the patient, and possibly additional trauma to the adjacent tissue. In addition to the pain and discomfort, the patient must be subjected to an additional time consuming and complicated surgical procedure with the attendant risks of surgery.

WO2012/018680 A1 describes methods and systems for generating a tissue pocket in a patient. US 2009/0138074 A1 describes decellularized extracellular matrix of conditioned body tissues and uses thereof. US 2010/0233235 A1 describes compositions and methods for preventing cardiac arrhythmia.

There is thus a need to provide medical devices that are configured for implantation in the body, and substantially reduce or eliminate the harsh biological responses associated with conventional implanted medical devices, including inflammation, infection and thrombogenesis.

It is therefore an object of the present invention to provide encasement structures that are configured to encase a medical device therein and that substantially reduce or eliminate the harsh biological responses associated with conventional implanted medical devices, including inflammation, inflection and thrombogenesis, when implanted in the body.

It is another object of the present invention to provide ECM encasement structures that are configured to encase a medical device therein, and effectively improve biological functions and/or promote modulated healing of adjacent tissue and the growth of new tissue when implanted in the body.

It is another object of the present invention to provide ECM encasement structures that are configured to encase a medical device therein and administer one or more pharmacological or therapeutic agents when implanted in the body.

It is yet another object of the present invention to provide medical devices that are configured for insertion or implantation in the body and exhibit enhanced biocompatibility and hemocompatibility when inserted or implanted therein.

### SUMMARY OF THE INVENTION

The present invention is directed to extracellular matrix (ECM) encasement structures for encasing medical devices in accordance with the claims.

The ECM encasement structures comprise a pocket or pouch that is configured to receive a medical device therein.

According to the invention, the medical device can comprise, without limitation, a pacemaker, defibrillator, synthetic heart valve, ventricular assist device, artificial heart, physiological sensor, catheter, and associated components, e.g., the electrical leads and lines associated therewith.

In a preferred embodiment, the ECM encasement structures are configured to encase an entire medical device.

In some embodiments, the ECM encasement structures are also configured to encase at least a portion of the medical device electrical leads.

In some embodiments of the invention, the ECM encasement structures include lead conduits that are configured to encase the medical device leads.

The ECM encasement structures comprise (or is constructed of) an ECM composition that includes at least one ECM material.

In a preferred embodiment, the ECM material referenced above comprises mammalian extracellular matrix tissue selected from the group comprising small intestine submucosa (SIS), urinary bladder submucosa (UBS), stomach submucosa (SS), epithelium of mesodermal origin, i.e. mesothelial tissue, dermal extracellular matrix, subcutaneous extracellular matrix, gastrointestinal extracellular matrix, i.e. large and small intestines, tissue surrounding growing bone, placental extracellular matrix, omamentum extracellular matrix, cardiac extracellular matrix, e.g., pericardium and/or myocardium, kidney extracellular matrix, pancreas extracellular matrix, lung extracellular matrix, and combinations thereof.

In some embodiments of the invention, the ECM material and, hence ECM encasement structures formed therefrom include at least one additional biologically active agent or composition, i.e. an agent that induces or modulates a physiological or biological process, or cellular activity, e.g., induces proliferation, and/or growth and/or regeneration of tissue.

In some embodiments of the invention, the biologically active agent comprises a growth factor.

In some embodiments of the invention, the biologically active component comprises chitosan.

In some embodiments, the ECM material and, hence ECM encasement structures formed therefrom include at least one pharmacological agent or composition (or drug), i.e. an agent or composition that is capable of producing a desired biological effect *in vivo,* e.g., stimulation or suppression of apoptosis, stimulation or suppression of an immune response, etc.

In some embodiments, the pharmacological agent or composition is selected from the group comprising antibiotics or antifungal agents, anti-viral agents, anti-pain agents, anesthetics, analgesics, steroidal anti-inflammatories, non-steroidal anti-inflammatories, anti-neoplastics, anti-spasmodics, modulators of cell-extracellular matrix interactions, proteins, hormones, enzymes and enzyme inhibitors, anticoagulants and/or antithrombic agents, DNA, RNA, modified DNA and RNA, NSAIDs, inhibitors of DNA, RNA or protein synthesis, polypeptides, oligonucleotides, polynucleotides, nucleoproteins, compounds modulating cell migration, compounds modulating proliferation and growth of tissue, and vasodilating agents.

In some embodiments of the invention, the pharmacological agent specifically comprises an anti-inflammatory agent or composition.

In some embodiments of the invention, the pharmacological agent comprises a statin, i.e. a *HMG-CoA* reductase inhibitor.

According to the invention, upon deployment of an encased medical device of the invention, i.e. an ECM encasement structure having a medical device therein modulated healing and regeneration of tissue structures with site-specific structural and functional properties are effectuated.

### BRIEF DESCRIPTION OF THE DRAWINGS

Further features and advantages will become apparent from the following and more particular description of the preferred embodiments of the invention, as illustrated in the accompanying drawings, and in which like referenced characters generally refer to the same parts or elements throughout the views, and in which:
FIGURE 1 is a perspective view of a biventricular (Bi-V) pacemaker;
FIGURE 2 is a perspective view of one embodiment of an ECM encasement structure having the Bi-V pacemaker shown in FIGURE 1 encased therein, in accordance with the invention;
FIGURE 3 is a perspective view of an ECM encasement structure, illustrating a folded pre-lamination configuration of an ECM pouch layer, in accordance with the invention;
FIGURE 4 is a front, partial sectional plan view of the ECM encasement structure shown in FIGURE 3, illustrating a laminated ECM pouch layer end, in accordance with the invention;
FIGURE 5 is a front, partial sectional plan view of another embodiment of an ECM encasement structure, in accordance with the invention;
FIGURES 6 and 7 are top plane views of further embodiments of ECM encasement structures, wherein the encasement structures include electrical lead conduits; in accordance with the invention;
FIGURE 8 is a perspective view of one embodiment of a medical device having an ECM composition coating thereon, in accordance with the invention; and
FIGURE 9 is a front, partial sectional plan view of the coated medical device shown in FIGURE 8, in accordance with the invention.

### DETAILED DESCRIPTION OF THE PREFERRED EMBODIMENT

The invention is defined by the accompanying claims.

It is also to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

As used in this specification and the appended claims, the singular forms "a, "an" and "the" include plural referents unless the content clearly dictates otherwise. Thus, for example, reference to "an active" includes two or more such actives and the like.

Further, ranges can be expressed herein as from "about" or "approximately" one particular value, and/or to "about" or "approximately" another particular value. When such a range is expressed, another embodiment includes from the one particular value and/or to the other particular value. Similarly, when values are expressed as approximations, by use of the antecedent "about" or "approximately", it will be understood that the particular value forms another embodiment. It will be further understood that the endpoints of each of the ranges are significant both in relation to the other endpoint, and independently of the other endpoint.

It is also understood that there are a number of values disclosed herein, and that each value is also herein disclosed as "about" or "approximately" that particular value in addition to the value itself. For example, if the value "10" is disclosed, then "approximately 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed then "less than or equal to 10" as well as "greater than or equal to 10" is also disclosed.

### Definitions

The term "medical device", as used herein, means and includes any device configured for insertion or implantation in the body of a warm blooded mammal, including humans and primates; avians; domestic household or farm animals, such as cats, dogs, sheep, goats, cattle, horses and pigs; laboratory animals, such as mice, rats and guinea pigs; fish; reptiles; zoo and wild animals; and the like. The term "medical device" thus includes, without limitation, a pacemaker, defibrillator, synthetic heart valve, ventricular assist device, artificial heart, physiological sensor, catheter, and associated components thereof, including electrical leads and lines associated therewith.

The terms "extracellular matrix", "ECM" and "ECM material" are used interchangeably herein, and mean and include a collagen-rich substance that is found in between cells in mammalian tissue. The invention uses decellularized ECM. According to the invention, the ECM material can be derived from a variety of mammalian tissue sources, including, without limitation, small intestine submucosa (SIS), urinary bladder submucosa (UBS), stomach submucosa (SS) epithelium of mesodermal origin, i.e. mesothelial tissue, dermal extracellular matrix, subcutaneous extracellular matrix, gastrointestinal extracellular matrix, i.e. large and small intestines, tissue surrounding growing bone, placental extracellular matrix, ornamentum extracellular matrix, cardiac extracellular matrix, e.g., pericardium and/or myocardium, kidney extracellular matrix, pancreas extracellular matrix, lung extracellular matrix, and combinations thereof. The ECM material can also comprise collagen from mammalian sources.

The terms "urinary bladder submucosa (UBS)", "small intestine submucosa (SIS)" and "stomach submucosa (SS)" also mean and include any UBS and/or SIS and/or SS material that includes the tunica mucosa (which includes the transitional epithelial layer and the tunica propria), submucosal layer, one or more layers of muscularis, and adventitia (a loose connective tissue layer) associated therewith.

The ECM material can also be derived from basement membrane of mammalian tissue/organs, including, without limitation, urinary basement membrane (UBM), liver basement membrane (LBM),

The ECM material can also be derived from other sources, including, without limitation, collagen from plant sources and synthesized extracellular matrices, i.e. cell cultures.

The term "angiogenesis", as used herein, means a physiologic process involving the growth of new blood vessels from pre-existing blood vessels.

The term "neovascularization', as used herein, means and includes the formation of functional vascular networks that can be perfused by blood or blood components. Neovascularization includes angiogenesis, budding angiogenesis, intussuceptive angiogenesis, sprouting angiogenesis, therapeutic angiogenesis and vasculogenesis.

The terms "biologically active agent" and "biologically active composition" are used interchangeably herein, and mean and include agent that induces or modulates a physiological or biological process, or cellular activity, e.g., induces proliferation, and/or growth and/or regeneration of tissue.

The terms "biologically active agent" and "biologically active composition" thus mean and include, without limitation, the following growth factors: platelet derived growth factor (PDGF), epidermal growth factor (EGF), transforming growth factor alpha (TGF-alpha), transforming growth factor beta (TGF-beta), fibroblast growth factor -2 (FGF-2), basic fibroblast growth factor (bFGF), vascular epithelial growth factor (VEGF), hepatocyte growth factor (HGF), insulin-like growth factor (IGF), nerve growth factor (NGF), platlet derived growth factor (PDGF), tumor necrosis factor alpha (TNA-alpha), and placental growth factor (PLGF).

The terms "biologically active agent" and "biologically active composition" also mean and include, without limitation, the following biologically active agents (referred to interchangeably herein as a "protein", "peptide" and "polypeptide"): collagen (types I-V), proteoglycans, glycosaminoglycans (GAGs), glycoproteins, growth factors, cytokines, cell-surface associated proteins, cell adhesion molecules (CAM), angiogenic growth factors, endothelial ligands, matrikines, cadherins, immuoglobins, fibril collagens, non-fibrallar collagens, basement membrane collagens, multiplexins, small-leucine rich proteoglycans, decorins, biglycans, fibromodulins, keratocans, lumicans, epiphycans, heparin sulfate proteoglycans, perlecans, agrins, testicans, syndecans, glypicans, serglycins, selectins, lecticans, aggrecans, versicans, neurocans, brevicans, cytoplasmic domain-44 (CD-44), macrophage stimulating factors, amyloid precursor proteins, heparins, chondroitin sulfate B (dermatan sulfate), chondroitin sulfate A, heparin sulfates, hyaluronic acids, fibronectins, tenascins, elastins, fibrillins, laminins, nidogen/enactins, fibulin I, finulin II, integrins, transmembrane molecules, thrombospondins, ostepontins, and angiotensin converting enzymes (ACE).

The terms "pharmacological agent", "active agent', "drug" and "active agent formulation" are used interchangeably herein, and mean and include an agent, drug, compound, composition of matter or mixture thereof, including its formulation, which provides some therapeutic, often beneficial, effect. This includes any physiologically or pharmacologically active substance that produces a localized or systemic effect or effects in animals, including warm blooded mammals, humans and primates; avians; domestic household or farm animals, such as cats, dogs, sheep, goats, cattle, horses and pigs; laboratory animals, such as mice, rats and guinea pigs; fish; reptiles; zoo and wild animals; and the like.

The terms "pharmacological agent", "active agent', "drug" and "active agent formulation" thus mean and include, without limitation, antibiotics, anti-arrhythmic agents, anti-viral agents, analgesics, steroidal anti-inflammatories, non-steroidal anti-inflammatories, anti-neoplastics, anti-spasmodics, modulators of cell-extracellular matrix interactions, proteins, hormones, growth factors, matrix metalloproteinases (MMPS), enzymes and enzyme inhibitors, anticoagulants and/or antithrombic agents, DNA, RNA, modified DNA and RNA, NSAIDs, inhibitors of DNA, RNA or protein synthesis, polypeptides, oligonucleotides, polynucleotides, nucleoproteins, compounds modulating cell migration, compounds modulating proliferation and growth of tissue, and vasodilating agents.

The terms "pharmacological agent", "active agent", "drug" and "active agent formulation" thus include, without limitation, atropine, tropicamide, dexamethasone, dexamethasone phosphate, betamethasone, betamethasone phosphate, prednisolone, triamcinolone, triamcinolone acetonide, fluocinolone acetonide, anecortave acetate, budesonide, cyclosporine, FK-506, rapamycin, ruboxistaurin, midostaurin, flurbiprofen, suprofen, ketoprofen, diclofenac, ketorolac, nepafenac, lidocaine, neomycin, polymyxin b, bacitracin, gramicidin, gentamicin, oyxtetracycline, ciprofloxacin, ofloxacin, tobramycin, amikacin, vancomycin, cefazolin, ticarcillin, chloramphenicol, miconazole, itraconazole, trifluridine, vidarabine, ganciclovir, acyclovir, cidofovir, ara-amp, foscarnet, idoxuridine, adefovir dipivoxil, methotrexate, carboplatin, phenylephrine, epinephrine, dipivefrin, timolol, 6-hydroxydopamine, betaxolol, pilocarpine, carbachol, physostigmine, demecarium, dorzolamide, brinzolamide, latanoprost, sodium hyaluronate, insulin, verteporfin, pegaptanib, ranibizumab, and other antibodies, antineoplastics, anti VGEFs, ciliary neurotrophic factor, brain-derived neurotrophic factor, bFGF, Caspase-1 inhibitors, Caspase-3 inhibitors, α-Adrenoceptors agonists, NMDA antagonists, Glial cell line-derived neurotrophic factors (GDNF), pigment epithelium-derived factor (PEDF), and NT-3, NT-4, NGF, IGF-2.

The terms "pharmacological agent", "active agent", "drug" and "active agent formulation" further mean and include the following Class I-Class V antiarrhythmic agents: (Class la) quinidine, procainamide and disopyramide; (Class Ib) lidocaine, phenytoin and mexiletine; (Class Ic) flecainide, propafenone and moricizine; (Class II) propranolol, esmolol, timolol, metoprolol and atenolol; (Class III) amiodarone, sotalol, ibutilide and dofetilide; (Class IV) verapamil and diltiazem) and (Class V) adenosine and digoxin.

The terms "pharmacological agent", "active agent", "drug" and "active agent formulation" further mean and include, without limitation, the following antiobiotics: aminoglycosides, cephalosporins, chloramphenicol, clindamycin, erythromycins, fluoroquinolones, macrolides, azolides, metronidazole, penicillins, tetracyclines, trimethoprim-sulfamethoxazole and vancomycin.

The terms "pharmacological agent", "active agent", "drug" and "active agent formulation" further include, without limitation, the following steroids: andranes (e.g., testosterone), cholestanes, cholic acids, corticosteroids (e.g., dexamethasone), estraenes (e.g., estradiol) and pregnanes (e.g., progesterone).

The terms "pharmacological agent", "active agent', "drug" and "active agent formulation" can further include one or more classes of narcotic analgesics, including, without limitation, morphine, codeine, heroin, hydromorphone, levorphanol, meperidine, methadone, oxycodone, propoxyphene, fentanyl, methadone, naloxone, buprenorphine, butorphanol, nalbuphine and pentazocine.

The terms "pharmacological agent", "active agent", "drug" and "active agent formulation" can further include one or more classes of topical or local anesthetics, including, without limitation, esters, such as benzocaine, chloroprocaine, cocaine, cyclomethycaine, dimethocaine/larocaine, piperocaine, propoxycaine, procaine/novacaine, proparacaine, and tetracaine/amethocaine. Local anesthetics can also include, without limitation, amides, such as articaine, bupivacaine, cinchocaine/dibucaine, etidocaine, levobupivacaine, lidocaine/lignocaine, mepivacaine, prilocaine, ropivacaine, and trimecaine. Local anesthetics can further include combinations of the above from either amides or esters.

The terms "pharmacological agent', "active agent', "drug" and "active agent formulation" can further include one or more classes of cytotoxic anti-neoplastic agents or chemotherapy agents, including, without limitation, alkylating agents, cisplatin, carboplatin, oxaliplatin, mechlorethamine, cyclophosphamide, chlorambucil, and ifosfamide.

Chemotherapy agents can also include, without limitation, antimetabolites, such as purine analogues, pyrimidine analogues and antifolates, plant alkaloids, such as vincristine, vinblastine, vinorelbine, vindesine, podophyllotoxin, etoposide and teniposide, taxanes, such as paclitaxel and docetaxel, topoisomerase inhibitors, such as irinotecan, topotecan, amsacrine, etoposide, etoposide phosphate and teniposide, cytotoxic antibiotics, such as actinomyocin, bleomycin, plicamycin, mytomycin and anthracyclines, such as doxorubicin, daunorubicin, valrubicin, idarubicin, epirubicin, and antibody treatments, such as abciximab, adamlimumab, alamtuzumab, basiliximab, belimumab, bevacizumab, brentuximab vedotin, canakinumab, cetuximab, certolizumab pego, daclizumab, denosumab, eculizumab, efalizumab, gemtuzumab, golimumab, ibritumomab tiuxetan, infliximab, ipilimumab, muromonab-CD3, natalizumab, ofatumumab, omalizumab, palivizumab, panitumumab, ranibizumab, rituximab, tocilizumab (atlizumab), tositumomab and trastuzumab.

The terms "anti-inflammatory" and "anti-inflammatory agent" are also used interchangeably herein, and mean and include a "pharmacological agent" and/or "active agent formulation', which, when a therapeutically effective amount is administered to a subject, prevents or treats bodily tissue inflammation i.e. the protective tissue response to injury or destruction of tissues, which serves to destroy, dilute, or wall off both the injurious agent and the injured tissues.

Anti-inflammatory agents thus include, without limitation, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, decanoate, deflazacort, delatestryl, depo-testosterone, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, mesterolone, methandrostenolone, methenolone, methenolone acetate, methylprednisolone suleptanate, momiflumate, nabumetone, nandrolone, naproxen, naproxen sodium, naproxol, nimazone, olsalazine sodium, orgotein, orpanoxin, oxandrolane, oxaprozin, oxyphenbutazone, oxymetholone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, stanozolol, sudoxicam, sulindac, suprofen, talmetacin, talniflumate, talosalate, tebufelone, tenidap, tenidap sodium, tenoxicam, tesicam, tesimide, testosterone, testosterone blends, tetrydamine, tiopinac, tixocortol pivalate, tolmetin, tolmetin sodium, triclonide, triflumidate, zidometacin, and zomepirac sodium.

The term "pharmacological composition', as used herein, means and includes a composition comprising a "pharmacological agent" and/or a "biologically active agent" and/or any additional agent or component identified herein.

The term "therapeutically effective", as used herein, means that the amount of the "pharmacological composition" and/or "pharmacological agent" and/or "biologically active agent" administered is of sufficient quantity to ameliorate one or more causes, symptoms, or sequelae of a disease or disorder. Such amelioration only requires a reduction or alteration, not necessarily elimination, of the cause, symptom, or sequelae of a disease or disorder.

The terms "prevent" and "preventing" are used interchangeably herein, and mean and include reducing the frequency or severity of a disease or condition. The term does not require an absolute preclusion of the disease or condition. Rather, this term includes decreasing the chance for disease occurrence.

The terms "treat" and "treatment" are used interchangeably herein, and mean and include medical management of a patient with the intent to cure, ameliorate, stabilize, or prevent a disease, pathological condition, or disorder. The terms include "active treatment", i.e. treatment directed specifically toward the improvement of a disease, pathological condition, or disorder, and "causal treatment", i.e. treatment directed toward removal of the cause of the associated disease, pathological condition, or disorder.

The terms "treat" and "treatment" further include "palliative treatment", i.e. treatment designed for the relief of symptoms rather than the curing of the disease, pathological condition, or disorder, "preventative treatment", i.e. treatment directed to minimizing or partially or completely inhibiting the development of the associated disease, pathological condition, or disorder, and "supportive treatment', i.e. treatment employed to supplement another specific therapy directed toward the improvement of the associated disease, pathological condition, or disorder.

The terms "patient" and "subject" are used interchangeably herein, and mean and include warm blooded mammals, humans and primates; avians; domestic household or farm animals, such as cats, dogs, sheep, goats, cattle, horses and pigs; laboratory animals, such as mice, rats and guinea pigs; fish; reptiles; zoo and wild animals; and the like.

The term "comprise" and variations of the term, such as "comprising" and "comprises," means "including, but not limited to" and is not intended to exclude, for example, other additives, components, integers or steps.

The following disclosure is provided to further explain in an enabling fashion the best modes of performing one or more embodiments of the present invention. The disclosure is further offered to enhance an understanding and appreciation for the inventive principles and advantages thereof, rather than to limit in any manner the invention. The invention is defined solely by the appended claims,

It is also understood that, although the present invention is described and illustrated in connection with a pacemaker, the invention is not limited to the noted medical device. Indeed, as stated above, the ECM encasement structures and compositions of the invention can also be employed to encase other medical devices, including without limitation, a defibrillator, synthetic heart valve, ventricular assist device, artificial heart, physiological sensor, catheter, and associated components thereof, including electrical leads and lines associated therewith.

As discussed above, the present invention is directed to extracellular matrix (ECM) encasement structures for encasing medical devices in accordance with the claims.

According to the invention, upon deployment of an ECM encasement structure having a medical device therein of the invention, modulated healing and regeneration of tissue structures with site-specific structural and functional properties are effectuated.

The phrase "modulated healing", as used herein, and variants of this language generally refer to the modulation (e.g., alteration, delay, retardation, reduction, etc.) of a process involving different cascades or sequences of naturally occurring tissue repair in response to localized tissue damage or injury, substantially reducing their inflammatory effect. Modulated healing, as used herein, includes many different biologic processes, including epithelial growth, fibrin deposition, platelet activation and attachment, inhibition, proliferation and/or differentiation, connective fibrous tissue production and function, angiogenesis, and several stages of acute and/or chronic inflammation, and their interplay with each other.

The ECM compositions of the invention are specifically formulated (or designed) to alter, delay, retard, reduce, and/or detain one or more of the phases associated with healing of damaged tissue, including, but not limited to, the inflammatory phase (e.g., platelet or fibrin deposition), and the proliferative phase.

"Modulated healing" refers to the ability of an ECM composition to alter a substantial inflammatory phase (e.g., platelet or fibrin deposition) at the beginning of the tissue healing process. As used herein, the phrase "alter a substantial inflammatory phase" refers to the ability of an ECM composition to substantially reduce the inflammatory response at an injury site.

In such an instance, a minor amount of inflammation may ensue in response to tissue injury, but this level of inflammation response, e.g., platelet and/or fibrin deposition, is substantially reduced when compared to inflammation that takes place in the absence of an ECM composition of the invention.

For example, the ECM compositions discussed herein have been shown experimentally to delay or alter the inflammatory response associated with damaged tissue, as well as excessive formation of connective fibrous tissue following tissue damage or injury. The ECM compositions have also been shown experimentally to delay or reduce fibrin deposition and platelet attachment to a blood contact surface following tissue damage.

"Modulated healing" refers to the ability of an ECM composition of the invention to induce host tissue proliferation, bioremodeling, including neovascularization, e.g., vasculogenesis, angiogenesis, and intussusception, and regeneration of tissue structures with site-specific structural and functional properties.

Accordingly, the ECM compositions discussed herein provide an excellent bioabsorbable cellular interface suitable for use with a medical device or surgical instrument.

As indicated above, the invention provides an ECM encasement structure which comprises an ECM based pocket or pouch that is configured to receive a medical device therein.

According to the invention, the encased medical device and associated components can comprise, without limitation, a pacemaker, defibrillator, synthetic heart valve, ventricular assist device, artificial heart, physiological sensor, catheter, and the electrical leads and lines associated therewith.

According to the invention, the entire medical device or a portion thereof can be encased in the ECM encasement structure. Thus, in some embodiments of the invention, the medical device housing and a portion of the device leads are encased in an ECM based pouch. In the noted embodiments, the device leads can also be coated with an ECM composition of the invention.

In some embodiments of the invention, the ECM encasement structure includes at least one lead conduit, more preferably, a plurality of lead conduits that are configured to encase the medical device leads.

The ECM encasement structure comprises (or is constructed of) an ECM composition that includes at least one ECM material (hereinafter "ECM pouch"). According to the invention, the ECM pouch can comprise various shapes and sizes to accommodate virtually all shapes and sizes of medical devices.

According to the invention, the ECM material can be derived from various mammalian tissue sources and methods for preparing same, such as disclosed in U.S. Pat. Nos. 7,550,004, 7,244,444, 6,379,710, 6,358,284, 6,206,931, 5,733,337 and 4,902,508 and U.S. Application No. 12/707,427;

The ECM material can also be sterilized via applicant's proprietary sterilization (i.e. novasterillis) process, as disclosed in Co-Pending U.S. Application No. 13/480205 (published as US2012302499).

In a preferred embodiment, the mammalian tissue sources include, without limitation, small intestine submucosa (SIS), urinary bladder submucosa (UBS), stomach submucosa (SS), epithelium of mesodermal origin, i.e. mesothelial tissue, dermal extracellular matrix, subcutaneous extracellular matrix, gastrointestinal extracellular matrix, i.e. large and small intestines, tissue surrounding growing bone, placental extracellular matrix, omamentum extracellular matrix, cardiac extracellular matrix, e.g., pericardium and/or myocardium, kidney extracellular matrix, pancreas extracellular matrix, lung extracellular matrix, and combinations thereof. The ECM material can also comprise collagen from mammalian sources.

The ECM material can also be derived from the same or different mammalian tissue sources, as disclosed in Co-Pending Application Nos. 13/033,053 (published as US2012034191) and 13, 033 102 (published as US2012016491).

As stated above, in some embodiments of the invention, the ECM material and, hence, ECM encasement structures formed therefrom include at least one additional biologically active agent or composition, i.e. an agent that induces or modulates a physiological or biological process, or cellular activity, e.g., induces proliferation, and/or growth and/or regeneration of tissue.

Suitable biologically active agents include any of the aforementioned biologically active agents, including, without limitation, proteins and growth factors.

In some embodiments, the ECM material and, hence, ECM encasement structures formed therefrom include at least one pharmacological agent or composition (or drug), i.e. an agent or composition that is capable of producing a desired biological effect *in vivo,* e.g., stimulation or suppression of apoptosis, stimulation or suppression of an immune response, etc.

Suitable pharmacological agents and compositions include any of the aforementioned agents, including, without limitation, antibiotics, anti-viral agents, analgesics, steroidal anti-inflammatories, non-steroidal anti-inflammatories, anti-neoplastics, anti-spasmodics, modulators of cell-extracellular matrix interactions, proteins, hormones, enzymes and enzyme inhibitors, anticoagulants and/or antithrombic agents, DNA, RNA, modified DNA and RNA, NSAIDs, inhibitors of DNA, RNA or protein synthesis, polypeptides, oligonucleotides, polynucleotides, nucleoproteins, compounds modulating cell migration, compounds modulating proliferation and growth of tissue, and vasodilating agents.

In some embodiments of the invention, the pharmacological agent comprises an anti-inflammatory agent.

In some embodiments of the invention, the pharmacological agent comprises a statin, i.e. a HMG-CoA reductase inhibitor. According to the invention, suitable statins include, without limitation, atorvastatin (Lipitor®), cerivastatin, fluvastatin (Lescol®), lovastatin (Mevacor®, Altocor®, Altoprev®), mevastatin, pitavastatin (Livalo®, Pitava®), pravastatin (Pravachol® , Selektine®, Lipostat®), rosuvastatin (Crestor®), and simvastatin (Zocor®, Lipex®). Several actives comprising a combination of a statin and another agent, such as ezetimbe/simvastatin (Vytorin®), are also suitable.

Applicant has found that the noted statins exhibit numerous beneficial properties that provide several beneficial biochemical actions or activities. The properties and beneficial actions are set forth in Applicant's Co-Pending Application Nos. 13/373,569, filed on September 24, 2012 and 13/782,024, filed on March 1, 2013 (published as US2014249623).

In some embodiments of the invention, the pharmacological agent comprises chitosan. As also set forth in detail in Co-Pending Application No. 13/573,569, chitosan also exhibits numerous beneficial properties that provide several beneficial biochemical actions or activities.

Additional suitable pharmacological agents and compositions that can be delivered within the scope of the invention are disclosed in Pat. Pub. Nos. 20070014874, 20070014873,20070014872,20070014871,20070014870,20070014869, and 20070014868;

According to the invention, the amount of a pharmacological agent added to an ECM composition of the invention will, of course, vary from agent to agent. For example, in one embodiment, wherein the pharmacological agent comprises dicloflenac (Voltaren®), the amount of dicloflenac included in the ECM composition is preferably in the range of 10 µg - 75 mg.

According to the invention, the biologically active and pharmacological agents referenced above can comprise any form. In some embodiments of the invention, the biologically active and pharmacological agents, e.g. simvastatin and/or chitosan, comprise microcapsules that provide delayed delivery of the agent contained therein.

As indicated above, upon deployment of an ECM encasement structure or a medical device (or instrument) coated with an ECM composition of the invention, modulated healing and regeneration of tissue structures with site-specific structural and functional properties is effectuated.

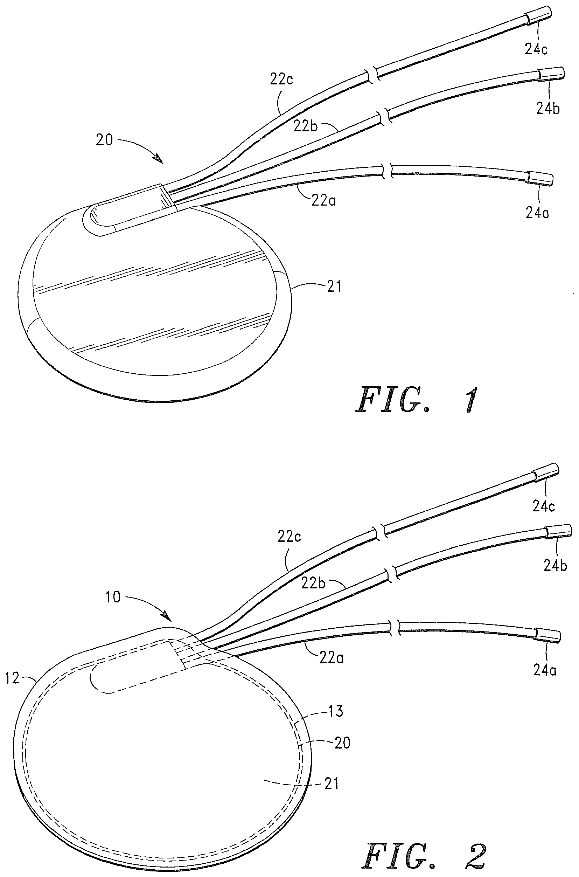

Referring now to Fig. 1, there is shown an exemplar implantable medical device; in this instance, a bi-ventricular (Bi-V) pacemaker 20, that can be encased by an ECM encasement structure of the invention. As is well known in the art and illustrated in Fig. 1, the Bi-V pacemaker 20 generally includes a pulse generator 21, electrical leads 22a, 22b, 22c and lead tips or electrodes 24a, 24b, 24c.

As is also well known in the art, the Bi-V pacemaker 20 is used to modulate the heart rate of a patient and prevent a life threatening heart dysfunction, e.g. arrhythmia.

The Bi-V pacemaker 20 is typically implanted transveniously in a patient, wherein two (2) electrical leads, i.e. leads 22a, 22b, are placed in a vein and guided to the right atrium and ventricle of the heart. The leads 22a, 22b are then attached to the heart muscle proximate the noted heart structures.

The third pacemaker lead, i.e., lead 22c, is also guided through a vein to the coronary sinus (i.e. a small vein on the back of the heart) and attached to the heart to pace the left ventricle.

Referring now to Fig. 2, there is shown a first embodiment of an ECM encasement structure of the invention 10, having the medical device 20 encased therein. As illustrated in Fig. 2, in this embodiment, the ECM encasement structure 10 is configured to encase the entire pacemaker 20 and a portion of the leads 22a, 22b, 22c, associated therewith.

The ECM encasement structure 10 comprises a pocket or pouch 12 having a cavity therein 13. The cavity 13 is sized and configured to receive and contain a medical device 20 therein.

The pouch 12 comprises at least one layer or sheet of encasement material constructed of an ECM composition of the invention. According to the invention, the pouch 12 can also include more than one layer of encasement material, e.g. two (2), three (3) encasement layers, etc. The encasement layers can also comprise the same material, i.e. ECM material or composition, or different materials or compositions.

In some embodiments of the invention, the ECM composition (or encasement layer(s)) and, hence, ECM encasement structure 10 formed therefrom include at least one additional biologically active agent or composition, i.e. an agent that induces or modulates a physiological or biological process, or cellular activity, e.g., induces proliferation, and/or growth and/or regeneration of tissue.

Suitable biologically active agents include any of the aforementioned biologically active agents, including, without limitation, proteins and growth factors.

In some embodiments, the ECM composition (or encasement layer(s)) and, hence, ECM encasement structure 10 formed therefrom include at least one pharmacological agent or composition (or drug), i.e. an agent or composition that is capable of producing a desired biological effect *in vivo,* e.g., stimulation or suppression of apoptosis, stimulation or suppression of an immune response, etc.

Suitable pharmacological agents and compositions include any of the aforementioned agents, including, without limitation, antibiotics, anti-viral agents, analgesics, and steroidal and non-steroidal anti-inflammatories.

According to the invention, the biologically active and pharmacological agents can be incorporated into the ECM composition (and/or material) and/or deposited on the outer surface of an outer encasement layer.

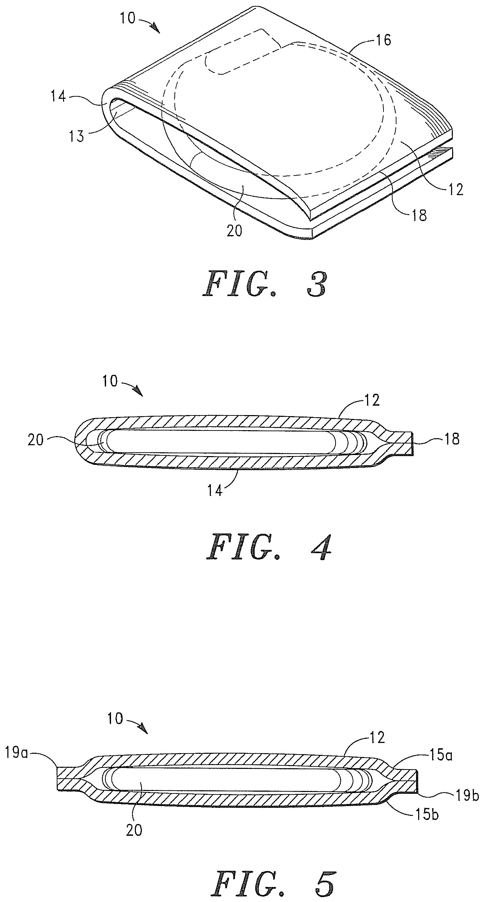

Referring now to Fig. 3, there is shown a perspective view of the ECM encasement structure 10, showing a folded pre-lamination configuration of the encasement layer (denoted "14"). As illustrated in Fig. 3, in the noted embodiment, the encasement layer 14 comprises a single sheet of encasement material. To form the pouch 12, the encasement layer 14 is folded over and laminated on the end 18 (see Fig. 4) and sides 16.

Referring now to Fig. 5, in some embodiments of the invention, the pouch 12 similarly comprises one encasement layer 14. However, in the noted embodiments, two (2) sheets of encasement material or layers 15a, 15b are employed to form the pouch 12. The layers 15a, 15b are laminated on both ends 19a, 19b, as shown in Fig 5, and sides.

According to the invention, the sides and ends of encasement layers of the invention can be laminated by various conventional means, such as stitching, including ECM stitches, stapled, adhesives. The encasement layers can also be laminated via microneedles and/or microneedle structures, such as disclosed in Co-Pending Application No. 13,686, 131 (published as US2014148897).

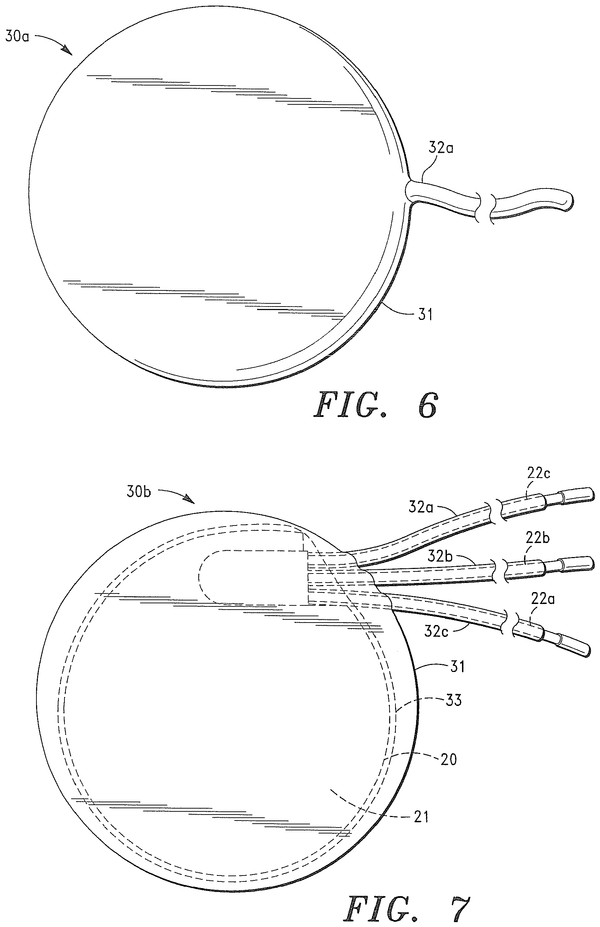

Referring now to Figs. 6 and 7, there is shown further embodiments ofECM encasement structures of the invention that are configured to encase medical devices, as well as the electrical leads associated therewith. Referring first to Fig. 6,
the ECM encasement structure 30a similarly comprises a pocket or pouch 31 having a cavity therein, such as shown in Fig. 2. The cavity is also sized and configured to receive and contain a medical device therein.

As illustrated in Fig. 6, the ECM encasement structure 30a further includes an integral lead conduit 32a that is configured to receive at least one medical device, e.g. pacemaker, lead therein.

Referring now to Fig. 7 there is shown another embodiment of an ECM encasement structure 30b, which similarly comprises a pocket or pouch 31 having a cavity 33 therein. As illustrated in Fig. 7, the cavity 33 is similarly designed and configured to contain a medical device; in this instance, pacemaker 20, therein.

In this embodiment, the ECM encasement structure 32b includes a plurality of lead conduits 32a, 32b, 32c that are configured to receive a plurality of device electrical leads, in this instance, pacemaker leads 22a, 22b, 22c, therein.

According to the invention, the ECM encasement structures 30a, 30b can comprise any of the aforementioned ECM compositions and/or materials in accordance with the claims. The ECM compositions and/or materials can similarly include any of the aforementioned biologically active pharmacological agents.

The lead conduits 32a, 32b, 32c can also be formed from the same ECM composition and/or material or a different ECM composition and/or material.

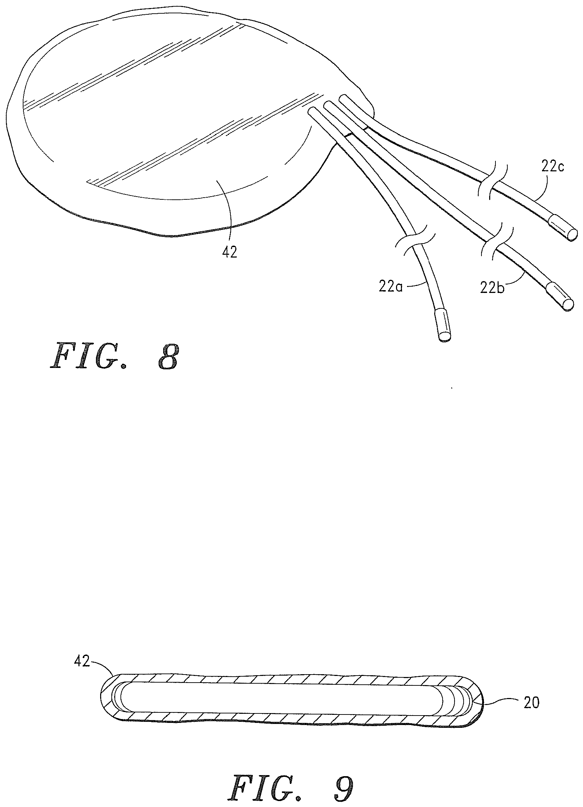

Referring now to Figs 8 and 9 which are present for illustration purposes only, there is shown a medical device, e.g. the Bi-V pacemaker, discussed above, having an ECM composition coating 42 disposed thereon. At least a portion of the medical device is coated with the ECM composition. coating.

The entire medical device and electrical leads associated therewith, e.g. leads 22a, 22b, 22c shown in Fig. 6, are coated with the ECM composition.

Various conventional means can be employed to form the coated biocompatible and hemocompatable medical device (and associated leads), including spray coating, dipping, etc..

As indicated above, upon deployment of an encased medical device of the invention, i.e. an ECM encasement structure modulated healing and regeneration of tissue structures with site-specific structural and functional properties are effectuated.

As will readily be appreciated by one having ordinary skill in the art, the present invention provides numerous advantages compared to prior art vascular endografts. Among the advantages are the following:
- The provision of encasement structures that are configured to encase a medical device therein and that substantially reduce or eliminate the harsh biological responses associated with conventional implanted medical devices, including inflammation, infection and thrombogenesis, when implanted in the body.
- The provision of ECM encasement structures that are configured to encase a medical device therein, and effectively improve biological functions and/or promote modulated healing of adjacent tissue and the growth of new tissue when implanted in the body.
- The provision of ECM encasement structures that are configured to encase a medical device therein and administer one or more pharmacological or therapeutic agents to a subject when implanted in his/her body.
- The provision of medical devices that are configured for insertion or implantation in the body and exhibit enhanced biocompatibility and hemocompatibility when inserted or implanted therein.

## Claims

1. A bioremodelable encasement structure, comprising:
a pouch formed from bioremodelable material, said pouch having an internal region configured to receive a device therein,
wherein the bioremodelable material comprises an extracellular matrix (ECM) composition in form of decellularized ECM, said ECM composition including one or more ECM scaffold component, wherein said one or more ECM scaffold component comprise mammalian tissue selected from the group consisting of small intestine submucosa (SIS), urinary bladder submucosa (UBS), urinary basement membrane (UBM), liver basement membrane (LBM), stomach submucosa (SS), collagen from animal sources, collagen from plant sources, synthesized extracellular matrix in cultures from cells, dermal extracellular matrix, subcutaneous extracellular matrix, large intestine extracellular matrix, placental extracellular matrix, ornamentum extracellular matrix, heart extracellular matrix, epithelium of mesodermal origin, gastrointestinal extracellular matrix, tissue surrounding growing bone, kidney extracellular matrix, pancreas extracellular matrix and lung extracellular matrix, wherein said pouch comprises a first sheet and a second sheet constructed from said one or more scaffold components;
wherein said first sheet and said second sheet are laminated at their respective edges by stitches, staples, adhesives, or microneedles to form the internal region and an opening configured to receive the medical device therethrough wherein the bioremodelable material does not comprise cells.

2. The encasement structure of Claim 1, wherein said ECM composition includes an additional bioactive agent component.

3. The encasement structure of Claim 2, wherein said bioactive agent component comprises a biologically active agent.

4. The encasement structure of Claim 3, wherein said biologically active agent comprises a growth factor selected from the group consisting of a platelet derived growth factor (PDGF), epidermal growth factor (EGF), transforming growth factor alpha (TGF-alpha), transforming growth factor beta (TGF-beta), fibroblast growth factor - 2 (FGF-2), basic fibroblast growth factor (bFGF), vascular epithelial growth factor (VEGF), hepatocyte growth factor (HGF), insulin-like growth factor (IGF), nerve growth factor (NGF), platlet derived growth factor (PDGF), tumor necrosis factor alpha (TNA-alpha), and placental growth factor (PLGF).

5. The encasement structure of Claim 3, wherein said biologically active agent comprises chitosan.

6. The encasement structure of Claim 2, wherein said bioactive agent component comprises a pharmacological agent selected from the group consisting of antibiotics, antifungal agents, anti-viral agents, anti-pain agents, anesthetics, analgesics, steroidal antiinflammatories, non-steroidal anti-inflammatories, anti-neoplastics, anti-spasmodics, modulators of cell-extracellular matrix interactions, proteins, hormones, enzymes and enzyme inhibitors, anticoagulants, antithrombic agents, DNA, RNA, modified DNA and RNA, NSAIDs, inhibitors of DNA, polypeptides, oligonucleotides, polynucleotides, nucleoproteins, and vasodilating agents.

7. The encasement structure of Claim 6, wherein said pharmacological agent comprises a statin selected from the group consisting of atorvastatin, cerivastatin, fluvastatin, lovastatin, mevastatin, pravastatin, pravastatin, rosuvastatin and simvastatin.

8. The encasement structure of Claim 1, wherein said device comprises a medical device selected from the group consisting of a pacemaker, ventricular assist device and physiological sensor.

9. encasement structure of Claim 1, wherein the pouch structure effectively improves biological functions by promoting tissue regeneration, modulated healing of damaged tissue, or growth of new tissue with site-specific structural and functional properties when implanted in a body of a warm-blooded animal.

## Patentansprüche

1. Bioremodellierbare Umhüllungsstruktur, umfassend:
einen Beutel, der aus bioremodellierbarem Material gebildet ist, wobei der Beutel einen Innenbereich aufweist, der konfiguriert ist, um eine Vorrichtung darin aufzunehmen,
wobei das bioremodellierbare Material eine extrazelluläre Matrix-(ECM-)Zusammensetzung in Form von dezellularisierter ECM umfasst, wobei die ECM-Zusammensetzung eine oder mehrere ECM-Gerüstkomponenten beinhaltet, wobei die eine oder die mehreren ECM-Gerüstkomponenten Säugetiergewebe umfassen, ausgewählt aus der Gruppe bestehend aus Dünndarmsubmukosa (SIS), Harnblasensubmukosa (UBS), Harnbasalmembran (UBM), Leberbasalmembran (LBM), Magensubmukosa (SS), Kollagen aus tierischen Quellen, Kollagen aus pflanzlichen Quellen, synthetisierter extrazellulärer Matrix in Kulturen aus Zellen, dermaler extrazellulärer Matrix, subkutaner extrazellulärer Matrix, extrazellulärer Matrix des Dickdarms, extrazellulärer Matrix der Plazenta, extrazellulärer Matrix von Ornamentum, extrazellulärer Matrix des Herzens, Epithel mesodermalen Ursprungs, extrazellulärer Matrix des Gastrointestinaltrakts, Gewebe um den wachsenden Knochen, extrazellulärer Matrix der Niere, extrazellulärer Matrix des Pankreas und extrazellulärer Matrix der Lunge, wobei der Beutel eine erste Lage und eine zweite Lage umfasst, die aus der einen oder den mehreren Gerüstkomponenten aufgebaut ist;
wobei die erste Lage und die zweite Lage an ihren jeweiligen Kanten durch Nähte, Heftklammern, Klebstoffe oder Mikronadeln laminiert sind, um den Innenbereich und eine Öffnung zu bilden, die konfiguriert ist, um die medizinische Vorrichtung dort hindurch aufzunehmen, wobei das bioremodellierbare Material keine Zellen umfasst.

2. Umhüllungsstruktur nach Anspruch 1, wobei die ECM-Zusammensetzung eine zusätzliche bioaktive Wirkstoffkomponente beinhaltet.

3. Umhüllungsstruktur nach Anspruch 2, wobei die bioaktive Wirkstoffkomponente einen biologisch aktiven Wirkstoff umfasst.

4. Umhüllungsstruktur nach Anspruch 3, wobei der biologisch aktive Wirkstoff einen Wachstumsfaktor umfasst, ausgewählt aus der Gruppe bestehend aus einem von Blutplättchen abgeleiteten Wachstumsfaktor (PDGF), epidermalem Wachstumsfaktor (EGF), transformierendem Wachstumsfaktor alpha (TGF-alpha), transformierendem Wachstumsfaktor beta (TGF-beta), Fibroblasten-Wachstumsfaktor - 2 (FGF-2), basischem Fibroblasten-Wachstumsfaktor (bFGF), vaskulärem epithelialen Wachstumsfaktor (VEGF), Hepatozyten-Wachstumsfaktor (HGF), insulinähnlichem Wachstumsfaktor (IGF), Nervenwachstumsfaktor (NGF), Thrombozyten-Wachstumsfaktor (PDGF), Tumornekrosefaktor alpha (TNA-Alpha) und Plazenta-Wachstumsfaktor (PLGF).

5. Umhüllungsstruktur nach Anspruch 3, wobei der biologisch aktive Wirkstoff Chitosan umfasst.

6. Umhüllungsstruktur nach Anspruch 2, wobei die bioaktive Wirkstoffkomponente einen pharmakologischen Wirkstoff umfasst, ausgewählt aus der Gruppe bestehend aus Antibiotika, antimykotischen Wirkstoffen, antiviralen Wirkstoffen, schmerzstillenden Wirkstoffen, Anästhetika, Analgetika, steroidalen Entzündungshemmern, nichtsteroidalen Entzündungshemmern, Antineoplastika, Antispasmodika, Modulatoren von zell-extrazellulären Matrix-Interaktionen, Proteinen, Hormonen, Enzymen und Enzyminhibitoren, Antikoagulanzien, Antithrombika, DNA, RNA, modifizierter DNA und RNA, NSAIDs, Inhibitoren von DNA, Polypeptiden, Oligonukleotiden, Polynukleotiden, Nukleoproteinen und gefäßerweiternden Wirkstoffen.

7. Umhüllungsstruktur nach Anspruch 6, wobei der pharmakologische Wirkstoff ein Statin umfasst, ausgewählt aus der Gruppe bestehend aus Atorvastatin, Cerivastatin, Fluvastatin, Lovastatin, Mevastatin, Pravastatin, Pravastatin, Rosuvastatin und Simvastatin.

8. Umhüllungsstruktur nach Anspruch 1, wobei die Vorrichtung eine medizinische Vorrichtung umfasst, ausgewählt aus der Gruppe bestehend aus einem Herzschrittmacher, einer ventrikulären Unterstützungsvorrichtung und einem physiologischen Sensor.

9. Umhüllungsstruktur nach Anspruch 1, wobei die Beutelstruktur biologische Funktionen wirksam verbessert, indem sie Geweberegeneration, modulierte Heilung von beschädigtem Gewebe oder Wachstum von neuem Gewebe mit ortsspezifischen strukturellen und funktionellen Eigenschaften fördert, wenn sie in einem Körper eines warmblütigen Tieres implantiert ist.

## Revendications

1. Structure d'enrobage bioremodelable, comprenant :
une poche formée d'un matériau bioremodelable, ladite poche ayant une région interne configurée pour y recevoir un dispositif,
dans laquelle le matériau bioremodelable comprend une composition de matrice extracellulaire (ECM) sous forme d'ECM décellularisée, ladite composition d'ECM comprenant un ou plusieurs composants d'échafaudage ECM, dans laquelle ledit un ou plusieurs composants d'échafaudage ECM comprennent un tissu de mammifère choisi dans le groupe constitué de la sous-muqueuse de l'intestin grêle (SIS), sous-muqueuse de la vessie (UBS), membrane basale urinaire (UBM), membrane basale du foie (LBM), sous-muqueuse de l'estomac (SS), collagène d'origine animale, collagène d'origine végétale, matrice extracellulaire synthétisée dans des cultures de cellules, matrice extracellulaire dermique, matrice extracellulaire sous-cutanée, matrice extracellulaire du gros intestin, matrice extracellulaire placentaire, matrice extracellulaire d'ornement, matrice extracellulaire cardiaque, épithélium d'origine mésodermique, matrice extracellulaire gastro-intestinale, tissu entourant l'os en croissance, matrice extracellulaire rénale, matrice extracellulaire pancréatique et matrice extracellulaire pulmonaire, dans laquelle ladite poche comprend une première feuille et une seconde feuille construite à partir desdits un ou plusieurs composants d'échafaudage ;
dans laquelle ladite première feuille et ladite seconde feuille sont stratifiées au niveau de leurs bords respectifs par des points, des agrafes, des adhésifs ou des micro-aiguilles pour former la région interne et une ouverture configurée pour recevoir le dispositif médical à travers celle-ci dans laquelle le matériau bioremodelable ne comprend pas de cellules.

2. Structure d'enrobage selon la revendication 1, dans laquelle ladite composition d'ECM comprend un composant d'agent bioactif supplémentaire.

3. Structure d'enrobage selon la revendication 2, dans laquelle ledit composant d'agent bioactif comprend un agent biologiquement actif.

4. Structure d'enrobage selon la revendication 3, dans laquelle ledit agent biologiquement actif comprend un facteur de croissance choisi dans le groupe constitué d'un facteur de croissance dérivé des plaquettes (PDGF), facteur de croissance épidermique (EGF), facteur de croissance transformant alpha (TGF-alpha), facteur de croissance transformant bêta (TGF-bêta), facteur de croissance des fibroblastes - 2 (FGF-2), facteur de croissance des fibroblastes basique (bFGF), facteur de croissance épithéliale vasculaire (VEGF), facteur de croissance des hépatocytes (HGF), facteur de croissance insulinomimétique (IGF), facteur de croissance des nerfs (NGF), facteur de croissance dérivé des plaquettes (PDGF), facteur de nécrose tumorale alpha (TNA-alpha) et facteur de croissance placentaire (PLGF).

5. Structure d'enrobage selon la revendication 3, dans laquelle ledit agent biologiquement actif comprend du chitosane.

6. Structure d'enrobage selon la revendication 2, dans laquelle ledit composant agent bioactif comprend un agent pharmacologique choisi dans le groupe constitué des antibiotiques, agents antifongiques, agents antiviraux, agents antidouleur, anesthésiques, analgésiques, anti-inflammatoires stéroïdiens, anti-inflammatoires non stéroïdiens, antinéoplasiques, antispasmodiques, modulateurs d'interactions cellule-matrice extracellulaire, protéines, hormones, enzymes et inhibiteurs d'enzymes, anticoagulants, agents antithrombiques, ADN, ARN, ADN et ARN modifiés, AINS, inhibiteurs d'ADN, polypeptides, oligonucléotides, polynucléotides, nucléoprotéines et agents vasodilatateurs.

7. Structure d'enrobage selon la revendication 6, dans laquelle ledit agent pharmacologique comprend une statine choisie dans le groupe constitué de l'atorvastatine, cérivastatine, fluvastatine, lovastatine, mévastatine, pravastatine, pravastatine, rosuvastatine et simvastatine.

8. Structure d'enrobage selon la revendication 1, dans laquelle ledit dispositif comprend un dispositif médical choisi dans le groupe constitué d'un stimulateur cardiaque, dispositif d'assistance ventriculaire et capteur physiologique.

9. Structure d'enrobage selon la revendication 1, dans laquelle la structure de poche améliore efficacement les fonctions biologiques en favorisant la régénération tissulaire, la cicatrisation modulée des tissus endommagés, ou la croissance de nouveaux tissus avec des propriétés structurelles et fonctionnelles spécifiques au site lorsqu'elle est implantée dans le corps d'un animal à sang chaud.
